# FASCICULE DE BREVET EUROPEEN

(11) **EP 2 646 406 B1**
(45) Date de publication et mention de la délivrance du brevet: **13.01.2016**
(21) Numéro de dépôt: 11802976.8
(22) Date de dépôt: 17.11.2011
(51) Int. Cl.: C07C 45/66, C07C 49/04, B01J 23/44

(54) **PROCÉDÉ POUR LA PRODUCTION DE DIBK**
VERFAHREN ZUR HERSTELLUNG VON DIBK
PROCESS FOR PRODUCING DIBK

(30) Priorité: 30.11.2010 FR 1059895
(43) Date de publication de la demande: 09.10.2013
(73) Titulaire: Rhodia Poliamida E Especialidades Ltda, Sao Paulo - SP (BR)
(72) Inventeur: ROSSO, Giovanni, CEP13087-460 Campinas SP (BR); MARTINS, Wilson, 13085-260 - Campinas (SP) (BR)
(74) Mandataire: Ridray, Annabelle
(86) Numéro de dépôt international: PCT/IB2011/002728
(87) Numéro de publication internationale: WO 2012/073082

(56) Documents cités:
- Yng-Long Hwang, Bedard, Thomas C.: "Ketones" In: "Ketones - Kirk-Othmer Encyclopedia of Chemical Technology", 1 janvier 2001 (2001-01-01), Wiley, XP055001266, vol. 14, pages 7-47, page 7, alinéa 6.2 * équations 1, 2 *
- DATABASE CASREACT [Online] CHEMICAL ABSTRACTS SERVICE, COLUMBUS, OHIO, US; 1984, HARADA, HARUHISA ET AL: "Direct conversion of diacetone alcohol to methyl", XP002644700, extrait de STN Database accession no. 105:6184 -& DATABASE WPI Week 198608 Thomson Scientific, London, GB; AN 1986-051928 XP002644701, & JP 61 005038 A (SUMITOMO CHEM IND KK) 10 janvier 1986 (1986-01-10)

## Description

La présente invention concerne un procédé de production de diisobutyl cétone (DIBK) consistant à mettre en présence de la triacétone dialcohol (TDA) et un catalyseur bifonctionnel capable d'effectuer une réaction de déshydratation et d'hydrogénation.

### ART ANTERIEUR

La diisobutyl cétone (DIBK) est un composé utilisé dans de nombreuses application industrielles. Il est notamment utilisé comme solvant pour certains polymères tels que la nitrocellulose et les résines epoxy. Il peut être utilisé pour des revêtements, peintures et encre notamment. Il est également utilisé comme solvant d'extraction dans l'industrie pharmaceutique.

Le DIBK est généralement produit en même temps que la méthylisobutyl cétone (MIBK) dans une réaction de condensation de l'acétone. Le document intitulé « Ketones », issu du Kirk-Othmer Encyclopedia of Chemical Technology décrit la préparation de DIBK à partir de 3 molécules d'acétone. Toutefois la conversion et la sélectivité en DIBK sont très faibles et largement insuffisantes.

### INVENTION

La demanderesse vient de mettre en évidence un nouveau procédé de production de la diisobutyl cétone (DIBK) dans lequel on met au moins en présence de la triacétone dialcohol (TDA) et un catalyseur bifonctionnel capable d'effectuer une réaction de déshydratation et d'hydrogénation. Ce procédé permet d'obtenir du DIBK avec une conversion et une sélectivité excellente.

Un catalyseur bifonctionnel est un type de catalyseur bien connu de la chimie et qui est capable de catalyser deux types de réactions, aussi connu en tant que catalyseur à double fonction. Le catalyseur bifonctionnel de l'invention permet la déshydratation en milieux acides ou basiques et permet l'hydrogénation notamment en présence d'un métal supporté sur le catalyseur.

On préfère notamment procéder à une déshydratation en milieu acide aidé par une haute température.

Le catalyseur bifonctionnel comprend comme premier composant un composé solide acide ou basique. Ce composé peut être un solide organique ou minéral du type alumine, zéolite, argile, céramique, phosphate, ou résine. A titre de support solide acide on peut citer notamment les résines d'acides sulfoniques, les résines carboxyliques, les résines phosphoriques, les oxydes minéraux tels que les zircones sulfatées, les argiles acides telles que les montmorillonites et les zéolites, tels que les H-ZSM5, et H-Y. A titre de support solide basique on peut citer les composés portant en surface des fonctions hydroxydes ou des fonctions aminées, des carbonates, des oxydes métalliques tels que les phosphate ou oxydes de lanthane, les argiles basiques telles que les hydroxydes double lamellaires (LDH).

Le système catalytique comprend comme second composant un composé A, métallique, alcalin ou métal alcalino-terreux. Comme composant métallique on peut notamment citer ceux à base de Cr, Co, Ni, Cu, Rh, Pd, Ir, Pt, Mo, W, Zn, P, As, Sb, Si, Ge, Sn, Al, Ga, Ti, Zr, Hf et/ou Au. Comme composant alcalin ou métal alcalino-terreux on peut notamment citer ceux à base de Li, Na, K, Rb, Cs, Be, Mg, Ca et/ou Sr. On préfère notamment Ni, Pd, Rh, et Ir. Ce composé peut être utilisé en tant que tel ou sous forme d'hydroxyde, d'oxyde ou de sel. Le métal est préférablement à l'état réduit pour son activité lors de l'hydrogénation.

Le catalyseur bifonctionnel comprend préférentiellement un composé métallique à base de Cr, Co, Ni, Cu, Rh, Pd, Ir, Pt, et/ou Au.

Les métaux du groupe du platine, en particulier le platine, le palladium, le rhodium et le ruthénium sont des catalyseurs particulièrement actifs, et qui agissent à basse température et faible pression en H₂.

Ces catalyseurs sont notamment décrit dans la demande US2003/0139629, le brevet US 6,008,416 et la publication «Pd supported on an Acidic resin...» Seki et al, Adv. Synth. Catal. 2008, 350, 691-705.

On peut notamment utiliser le composé A, métallique, alcalin ou alcalino-terreux, dans des proportions comprises entre 0,001 % et 30 % en poids, plus préférentiellement entre 0,01 % et 10 % en poids, par rapport au poids du composé solide acide ou basique.

Selon un objet préféré de l'invention, le système catalytique comprend un composé solide acide ou basique sur lequel le composé A décrit ci-dessus est supporté en surface. On peut par exemple citer les catalyseurs Amberlyst(R) CH28. Il est également possible que le système catalytique comprenne un composé solide acide ou basique et un composé A supporté sur un solide inerte vis-à-vis de la réaction.

Le catalyseur peut être mis sur un lit fixe ou bien être mis en suspension sous agitation dans le réacteur.

La proportion de catalyseur peut varier entre 0,01 % et 60 % en poids par rapport au poids du TDA, préférentiellement entre 0,1 % et 20 % en poids, plus préférentiellement entre 1 % et 10 % en poids.

Le milieu réactionnel comprend notamment du TDA, et éventuellement d'autres composés, comme un ou plusieurs solvants par exemple.

Le milieu réactionnel peut éventuellement comprendre un ou plusieurs solvants, tells que des solvants polaires ou apolaires, protique ou aprotiques, particulièrement les solvants protiques polaires, les solvants aprotiques polaires et les solvants aprotiques apolaires. On préfère notamment les alcools, tels que le méthanol, l'éthanol et l'isopropanone.

Le milieu réactionnel comprend préférentiellement de 30 à 70 % en poids de TDA et de 30 à 70 % en poids de DAA, plus particulièrement de 40 à 60 % en poids de TDA et de 40 à 60 % en poids de DAA.

Dans ce cas là, on procède ainsi à la fabrication de DIBK à partir du TDA et à la fabrication du MIBK à partir du DAA.

La présente invention concerne ainsi également un procédé pour la production de DIBK et de MIBK par mise en présence de TDA et DAA avec le catalyseur bifonctionnel de la présente invention.

Le milieu réactionnel peut être issu d'une unité de production de DAA, directement ou indirectement ; plus spécifiquement du pieds de la colonne de distillation d'une unité de production de DAA, successivement à la condensation catalysée de l'acétone. Il est parfaitement possible d'augmenter la concentration en TDA du milieu issu d'une unité de production de DAA, ou d'éliminer certaines impuretés, par distillation ou cristallisation par exemple.

Préférentiellement, la réaction est effectuée à une température comprise entre 10°C et 200°C, plus préférentiellement entre 30°C et 150°C. Il est notamment observé que la déshydratation du TDA est accrue par l'effet synergique de la catalyse acide du catalyseur bifonctionnel et les hautes températures.

Préférentiellement, la réaction est effectuée à une pression comprise entre 1 et 100 bar, plus préférentiellement entre 3 et 25 bar, encore plus préférentiellement entre 8 et 15 bar. Une telle pression peut être obtenue par ajout alimentation au réacteur d'hydrogène pur ou d'un mélange d'hydrogène et d'un gaz inerte, tel que par exemple l'azote ou l'argon. La pression partielle en hydrogène peut être maintenue par purge du ciel gazeux en contrôlant la teneur en hydrogène.

Le procédé selon l'invention peut être réalisé en continu ou en discontinu, préférentiellement en phase liquide. Le temps de séjour de la réaction peut notamment être de 5 à 300 minutes.

La réaction peut être effectuée dans un réacteur de tout type, notamment dans un tube réactionnel monté verticalement. Plusieurs réacteurs mettant en oeuvre le procédé de l'invention peuvent être mis en série.

On préfère notamment introduire dans un réacteur de la triacétone dialcool (TDA) et la mettre en présence avec un catalyseur bifonctionnel capable d'effectuer une réaction de déshydratation et d'hydrogénation et démarrer ensuite la réaction de synthèse de la diisobutyl cétone (DIBK). On peut notamment démarrer la réaction par pressurisation, notamment par l'hydrogène, et/ou mise en température.

Il est possible de procéder à une ou plusieurs étapes de purification, notamment par distillation, du produit obtenu après l'étape réactionnelle, par exemple pour récupérer le ou les réactifs.

Un langage spécifique est utilisé dans la description de manière à faciliter la compréhension du principe de l'invention. Il doit néanmoins être compris qu'aucune limitation de la portée de l'invention n'est envisagée par l'utilisation de ce langage spécifique. Des modifications, améliorations et perfectionnements peuvent notamment être envisagés par une personne au fait du domaine technique concerné sur la base de ses propres connaissances générales.

Le terme et/ou inclut les significations et, ou, ainsi que toutes les autres combinaisons possibles des éléments connectés à ce terme.

D'autres détails ou avantages de l'invention apparaîtront plus clairement au vu des exemples donnés ci-dessous uniquement à titre indicatif.

### PARTIE EXPERIMENTALE

### Exemple 1: Réaction en discontinu

Dans un réacteur agité de 100 ml, on effectue une charge de 7,01 g de catalyseur solide acide à base de résine sulfonique comportant du palladium et de 70,05 g d'un mélange comprenant 50 % en poids de TDA et 50 % en poids de DAA.

Ensuite, l'équipement est fermé, pressurisé à l'hydrogène, et chauffé jusqu'à une température de 120°C et une pression de 10 bar, pour une durée de 4 heures. En fin de réaction, la conversion du TDA atteint 100 % et la sélectivité du TDA en DIBK est de 85 %.

On observe par ailleurs la présence de MIBK. En fin de réaction, la conversion du DAA atteint 100 % et la sélectivité du DAA en MIBK est de 92 %.

La sélectivité d'une réaction chimique spécifie la quantité de produit désiré formé par rapport au nombre de moles consommées du réactif limitant. Elle indique si plusieurs réactions se produisent en parallèle, conduisant à des sous-produits non désirés, ou si la réaction recherchée est la seule à consommer du réactif. On observe ainsi une excellente sélectivité en DIBK dans le cadre du procédé de la présente invention.

### Exemple 2: comparaison

Dans un réacteur agité de 100 ml, on effectue une charge de 7,01 g de catalyseur solide acide à base de résine sulfonique comportant du palladium et de 70,04 g de d'acétone. Ensuite, l'équipement est fermé, pressurisé à l'hydrogène, et chauffé jusqu'à une température de 120°C et une pression de 10 bar, pour une durée de 3 heures. En fin de réaction, la conversion atteint 64 % et la sélectivité en DIBK est de 3 % uniquement.

## Revendications

1. Procédé de production de la diisobutyl cétone (DIBK) dans lequel on met au moins en présence de la triacétone dialcohol (TDA) et un catalyseur bifonctionnel capable d'effectuer une réaction de déshydratation et d'hydrogénation.

2. Procédé selon la revendication 1, **caractérisé en ce que** le catalyseur bifonctionnel comprend un composé solide acide ou basique du type alumine, zéolite, argile, céramique, phosphate, argile ou résine.

3. Procédé selon la revendication 2, **caractérisé en ce que** le composé solide acide est choisi dans le groupe comprenant les résines d'acides sulfoniques, les résines carboxyliques, les résines phosphoriques, les oxydes minéraux tels que les zircones sulfatées, les argiles acides telles que les montmorillonites et les zéolites, tels que les H-ZSM5, et H-Y.

4. Procédé selon la revendication 2, **caractérisé en ce que** le composé solide basique est choisi dans le groupe comprenant les composés portant en surface des fonctions hydroxydes ou des fonctions aminées, des carbonates, des oxydes métalliques tels que les phosphate ou oxydes de lanthane, les argiles basiques telles que les hydroxydes double lamellaires (LDH).

5. Procédé selon l'une quelconque des revendications 1 à 4, **caractérisé en ce que** le catalyseur bifonctionnel comprend comme second composant un composé A métallique, alcalin ou métal alcalino-terreux.

6. Procédé selon l'une quelconque des revendications 1 à 5, **caractérisé en ce que** le catalyseur bifonctionnel comprend un composé métallique à base de Cr, Co, Ni, Cu, Rh, Pd, Ir, Pt, et/ou Au.

7. Procédé selon l'une quelconque des revendications 5 à 6, **caractérisé en ce que** le composé A est présent dans des proportions comprises entre 0,001 % et 30 % en poids, plus préférentiellement entre 0,01 % et 10 % en poids, par rapport au poids du composé solide acide ou basique.

8. Procédé selon l'une quelconque des revendications 1 à 7, **caractérisé en ce que** le catalyseur comprend un composé solide acide ou basique sur lequel le composé métallique est supporté en surface.

9. Procédé selon l'une quelconque des revendications 1 à 8, **caractérisé en ce que** le milieu réactionnel comprend au moins du TDA et un solvant.

10. Procédé selon l'une quelconque des revendications 1 à 9, **caractérisé en ce que** le milieu réactionnel comprend de 30 à 70 % en poids de TDA et de 30 à 70 % en poids de DAA.

11. Procédé selon l'une quelconque des revendications 1 à 10, **caractérisé en ce que** la réaction est effectuée à une température comprise entre 10°C et 200°C.

12. Procédé selon l'une quelconque des revendications 1 à 11, **caractérisé en ce que** la réaction est effectuée à une pression comprise entre 1 et 100 bar.

## Patentansprüche

1. Verfahren zur Herstellung von Diisobutylketon (DIBK), wobei mindestens Triacetondialkohol (TDA) und ein bifunktioneller Katalysator, der geeignet ist, eine Reaktion zur Dehydratation und zur Hydrogenation auszuführen, in Kontakt gebracht werden.

2. Verfahren nach Anspruch 1, **dadurch gekennzeichnet, dass** der bifunktionelle Katalysator eine saure oder basische feste Verbindung vom Typ Aluminiumoxid, Zeolith, Ton, Keramik, Phosphat, Ton oder Harz umfasst.

3. Verfahren nach Anspruch 2, **dadurch gekennzeichnet, dass** die saure feste Verbindung ausgewählt ist aus der Gruppe umfassend Sulfonsäureharze, Carboxylharze, Phosphorsäureharze, mineralische Oxide, wie beispielsweise sulfatierte Zirkone, saure Tone, wie beispielsweise Montmorillonite und Zeolithe, wie beispielsweise H-ZSM5 und H-Y.

4. Verfahren nach Anspruch 2, **dadurch gekennzeichnet, dass** die basische feste Verbindung aus der Gruppe ausgewählt ist, die Verbindungen umfasst, die an der Oberfläche Hydroxidfunktionen oder Aminofunktionen, Carbonate, Metalloxide, wie beispielsweise Lanthanphosphate oder Lanthanoxide, basische Tone, wie beispielsweise Lamellar Double Hydroxide (LDH), tragen.

5. Verfahren nach einem der Ansprüche 1 bis 4, **dadurch gekennzeichnet, dass** der bifunktionelle Katalysator als zweiten Bestandteil eine metallische, alkalische oder erdalkalimetallische Verbindung A umfasst.

6. Verfahren nach einem der Ansprüche 1 bis 5, **dadurch gekennzeichnet, dass** der bifunktionelle Katalysator eine metallische Verbindung auf der Basis von Cr, Co, Ni, Cu, Rh, Pd, Ir, Pt und/oder Au umfasst.

7. Verfahren nach einem der Ansprüche 5 bis 6, **dadurch gekennzeichnet, dass** die Verbindung A in Anteilen im Bereich zwischen 0,001 Gew.-% und 30 Gew.-%, stärker bevorzugt zwischen 0,01 Gew.-% und 10 Gew.-%, bezogen auf das Gewicht der sauren oder basischen festen Verbindung, vorhanden ist.

8. Verfahren nach einem der Ansprüche 1 bis 7, **dadurch gekennzeichnet, dass** der Katalysator eine basische oder saure feste Verbindung umfasst, auf der die metallische Verbindung an der Oberfläche getragen wird.

9. Verfahren nach einem der Ansprüche 1 bis 8, **dadurch gekennzeichnet, dass** das Reaktionsmedium mindestens TDA und ein Lösungsmittel umfasst.

10. Verfahren nach einem der Ansprüche 1 bis 9, **dadurch gekennzeichnet, dass** das Reaktionsmedium 30 bis 70 Gew.-% TDA und 30 bis 70 Gew.-% DAA umfasst.

11. Verfahren nach einem der Ansprüche 1 bis 10, **dadurch gekennzeichnet, dass** die Reaktion bei einer Temperatur im Bereich zwischen 10 °C und 200 °C durchgeführt wird.

12. Verfahren nach einem der Ansprüche 1 bis 11, **dadurch gekennzeichnet, dass** die Reaktion bei einem Druck im Bereich zwischen 1 und 100 bar durchgeführt wird.

## Claims

1. Process for the production of diisobutyl ketone (DIBK), in which at least triacetone dialcohol (TDA) and a bifunctional catalyst capable of carrying out a dehydration and hydrogenation reaction are brought together.

2. Process according to Claim 1, **characterized in that** the bifunctional catalyst comprises an acidic or basic solid compound of the alumina, zeolite, clay, ceramic, phosphate, clay or resin type.

3. Process according to Claim 2, **characterized in that** the acidic solid compound is selected from the group consisting of sulfonic acid resins, carboxylic resins, phosphoric resins, inorganic oxides, such as sulfated zirconias, acidic clays, such as montmorillonites, and zeolites, such as H-ZSM5 and H-Y.

4. Process according to Claim 2, **characterized in that** the basic solid compound is selected from the group consisting of the compounds carrying, at the surface, hydroxide functional groups or amine functional groups, carbonates, metal oxides, such as lanthanum phosphates or oxides, or basic clays, such as lamellar double hydroxides (LDHs).

5. Process according to any one of Claims 1 to 4, **characterized in that** the bifunctional catalyst comprises, as second component, a metal, alkali metal or alkaline earth metal compound A.

6. Process according to any one of Claims 1 to 5, **characterized in that** the bifunctional catalyst comprises a metal compound based on Cr, Co, Ni, Cu, Rh, Pd, Ir, Pt and/or Au.

7. Process according to either one of Claims 5 and 6, **characterized in that** the compound A is present in proportions of between 0.001% and 30% by weight, more preferably between 0.01% and 10% by weight, with respect to the weight of the acidic or basic solid compound.

8. Process according to any one of Claims 1 to 7, **characterized in that** the catalyst comprises an acidic or basic solid compound on which the metal compound is supported at the surface.

9. Process according to any one of Claims 1 to 8, **characterized in that** the reaction medium comprises at least TDA and a solvent.

10. Process according to any one of Claims 1 to 9, **characterized in that** the reaction medium comprises from 30 to 70% by weight of TDA and from 30 to 70% by weight of DAA.

11. Process according to any one of Claims 1 to 10, **characterized in that** the reaction is carried out at a temperature of between 10°C and 200°C.

12. Process according to any one of Claims 1 to 11, **characterized in that** the reaction is carried out at a pressure of between 1 and 100 bar.
